(19) **Europäisches Patentamt / European Patent Office / Office européen des brevets**

(11) **EP 2 324 854 A1**

(12) # EUROPEAN PATENT APPLICATION
published in accordance with Art. 153(4) EPC

(43) Date of publication:
**25.05.2011 Bulletin 2011/21**

(21) Application number: **09806746.5**

(22) Date of filing: **13.08.2009**

(51) Int Cl.:
**A61K 47/04** *(2006.01)*  **A61K 9/70** *(2006.01)*
**A61K 31/192** *(2006.01)*  **A62B 18/02** *(2006.01)*
**A62B 23/02** *(2006.01)*  **B01J 20/20** *(2006.01)*
**B01J 20/28** *(2006.01)*  **C01B 31/02** *(2006.01)*

(86) International application number:
**PCT/JP2009/064289**

(87) International publication number:
**WO 2010/018855 (18.02.2010 Gazette 2010/07)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **14.08.2008 JP 2008208914**
**02.10.2008 JP 2008257132**
**03.08.2009 JP 2009180534**

(71) Applicant: **Sony Corporation**
**Tokyo 108-0075 (JP)**

(72) Inventors:
• **TABATA, Seiichiro**
  **Tokyo 108-0075 (JP)**
• **YAMADA, Shinichiro**
  **Tokyo 108-0075 (JP)**
• **HORIE, Takeshi**
  **Tokyo 108-0075 (JP)**
• **NOGUCHI, Tsutomu**
  **Tokyo 108-0075 (JP)**

(74) Representative: **Müller - Hoffmann & Partner Patentanwälte**
**Innere Wiener Strasse 17**
**81667 München (DE)**

(54) **DRUG SUSTAINED RELEASE AGENT, ADSORBENT, FUNCTIONAL FOOD, MASK, AND ADSORPTIVE SHEET**

(57) Provided is a drug sustained-release agent including a carbon material (porous carbon material) which has an inverse opal structure. The drug sustained-release agent includes a porous carbon material which has spherical pores having an average diameter of $1 \times 10^{-9}$ to $1 \times 10^{-5}$ m and arrayed three-dimensionally and which has a surface area of $3 \times 10^2$ m²/g. Or, the drug sustained-release agent includes a porous carbon material in which pores are arrayed in an arrangement corresponding to a crystal structure on a macroscopic basis. Or, the drug sustained-release agent includes a porous carbon material in which pores are arrayed at a surface thereof in an arrangement corresponding to the (111) plane orientation of a face-centered cubic structure on a macroscopic basis.

FIG.1

EP 2 324 854 A1

## Description

Technical Field

The present invention relates to a drug sustained-release agent having a porous carbon material, an adsorbent having a porous carbon material, and a functional food, a mask and an adsorption sheet which use such an adsorbent.

Background Art

In recent years, carbon materials having various nanostructures, for example, carbon materials having regular (ordered) fine structures of carbon nanotube, carbon nanohorn, zeolite, mesoporous silica, etc. have been attracting much attention. In addition, materials having the so-called inverse opal structure which are synthesized by using, as mold, a colloidal crystal body composed of monodisperse silica particulates, polystyrene particulates or the like have also been drawing attention. Hitherto, materials having the inverse opal structure which are composed of carbon, titania, silica, tin oxide, polymer material or the like have been reported, and the possibility of their various applications have been investigated. For example, carbon materials having the inverse opal structure are reported in "Carbon Structures with Three-Dimensional Periodicity at Optical Wavelengths," Anvar A. Zakhidov, et. al., Science, No. 282, 1998, pp 897 (Non-Patent Document 1), and their uses for lithium ion secondary cells, electric double layer capacitors, fuel cells or as an optical material utilizing structural color, and so on are investigated.

Besides, for example, Japanese Patent Laid-open No. 2005-262324 discloses a porous carbon material with pores having a three-dimensional regularity wherein the pores are arrayed in an arrangement such as to constitute a crystal structure on a macroscopic basis, a porous carbon material with pores having a three-dimensional regularity wherein the pores are arrayed in the (1, 1, 1) plane orientation of a face-centered cubic lattice, and, further, a method of manufacturing these porous carbon materials.

Meanwhile, for patients having a hepatic disease or renal disease, removal of toxic substance by hemodialysis is carried out. However, hemodialysis not only needs special equipment and a technician but also imposes great physical and/or mental burden on the patients. Under such a background, oral active carbon adsorbents such as Kremezin which are high in safety for living bodies and in stability have been attracting attention (see Japanese Patent Publication No. Sho 62-11611). Besides, antiobestic drug, antidiabetic drug, inflammatory bowel disease drug, adsorbent for purine bodies, etc. which use active carbon have also been proposed, and application and research and development of active carbon in medical fields have been advanced widely.

Or, on the other hand, for a drug to act effectively in a living body, it is desirable to cause an appropriate quantity of the drug to act over an appropriate time. For realizing this, in addition, it is preferable to use a carrier by which release rate of a drug can be controlled. When a drug is adsorbed on such a carrier, a constant quantity of the drug can be released continuously. Such a carrier-drug composite body can, for example, be used as a percutaneous drug having a percutaneous absorption localized action for transfer of the drug through a skin or as an oral drug. The carrier is composed of a material being nontoxic and resistant to chemicals, for example, an inorganic material such as carbon, alumina, silica, etc. or an organic material such as cellulose, polyethylene oxide, etc. Meanwhile, in recent years, there have been several reports of examples of use of a carbon material as a carrier (see, for example, Japanese Patent Laid-open No. 2005-343885). In addition, there have been reports concerning sustained release of a fertilizer by use of active carbon (see, for example, Japanese Patent No. 3694305).

Prior Art Documents

Patent Documents

Patent Document 1: Japanese Patent Laid-Open No. 2005-262324
Patent Document 2: Japanese Patent Publication No. Sho 62-11611
Patent Document 3: Japanese Patent Laid-Open No. 2005-343885
Patent Document 4: Japanese Patent No. 3694305

Non-Patent Document

Non-Patent Document 1: "Carbon Structures with Three-Dimensional Periodicity at Optical Wavelengths," Anvar A.

Zakhidov, et. al., Science, No. 282, 1998, pp 897

Summary of the Invention

Technical Problem

Meanwhile, in the above-mentioned Non-Patent Document 1, there is no description about application of the carbon materials having the inverse opal structure as a drug sustained-release agent or an adsorbent. Besides, in Japanese Patent Laid-Open No. 2005-262324, it is described that the porous carbon material is useful as an electrode material for capacitors, lithium ion cells, fuel cells, etc., as various conductive materials, or as an optical material for selective reflection at a specified wavelength. In this document, however, there is no description about application of the porous carbon material to drug sustained-release agent, adsorbent, or functional food, or in other fields.

Accordingly, it is an object of the present invention to provide a drug sustained-release agent and an adsorbent which have a carbon material (porous carbon material) having the inverse opal structure, a functional food using such a porous carbon material, and a mask and an adsorption sheet which use such an adsorbent.

Technical Solution

A drug sustained-release agent (a carrier-drug composite body capable of appropriately controlling the release rate of a drug), or an adsorbent for adsorbing an organic matter thereon, or an adsorbent for medical use, an adsorbent for oral administration, or an adsorbent for adsorbing an allergen thereon, according to a first embodiment of the present invention for attaining the above object has a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g, preferably not less than $1 \times 10^3$ m$^2$/g. If the average diameter of the pores is less than $1 \times 10^{-9}$ m (1 nm), the pores are too small and no conspicuous difference in characteristics can be seen between the porous carbon material and the bulk carbon material. On the other hand, if the average diameter of the pores exceeds $1 \times 10^{-5}$ m (10 $\mu$m), the pores are generally too large and the porous carbon material might be lowered in mechanical strength. Besides, if the surface area is less than $3 \times 10^2$ m$^2$/g, characteristic properties of the porous carbon material might be unsatisfactory.

A drug sustained-release agent, or an adsorbent for adsorbing an organic matter thereon, or an adsorbent for medical use, an adsorbent for oral administration, or an adsorbent for adsorbing an allergen thereon, according to a second embodiment of the present invention for attaining the above object has a porous carbon material in which pores are arrayed in an arrangement corresponding to a crystal structure on a macroscopic basis.

A drug sustained-release agent, or an adsorbent for adsorbing an organic matter thereon, or an adsorbent for medical use, an adsorbent for oral administration, or an adsorbent for adsorbing an allergen thereon, according to a third embodiment of the present invention for attaining the above object has a porous carbon material in which pores are arrayed at a surface thereof in an arrangement corresponding to the (111) plane orientation of a face-centered cubic structure on a macroscopic basis. Meanwhile, when the porous carbon material is cut along a virtual plane parallel to the surface thereof, in the virtual cutting plane the pores are arrayed in an arrangement corresponding to the (111) plane orientation of a face-centered cubic structure.

In the drug sustained-release agents according to the first to third embodiments of the present invention, the adsorbents for adsorbing an organic matter thereon according to the first to third embodiment of the invention, the adsorbents for medical use according to the first to third embodiment of the invention, the adsorbents for oral administration according to the first to third embodiment of the invention, the adsorbents for adsorbing an allergen thereon according to the first to third embodiment of the invention, functional foods according to the first to third embodiment of the invention which are to be described later, masks according to the first to third embodiment of the invention which are to be described later, and adsorption sheets according to the first to third embodiment of the invention which are to be described later, it is preferable that a surface of the porous carbon material has undergone a chemical treatment or molecular modification. Examples of the chemical treatment include a treatment in which carboxyl groups are formed on the surface by a nitric acid treatment. In addition, an activating treatment with water vapor, oxygen, alkali or the like may be performed, whereby various functional groups such as hydroxyl group, carboxyl group, ketone group, ester group, etc. can be produced on the surface of the porous carbon material. Further, the porous carbon material may be put into chemical reaction with a chemical species or protein having a group capable of reacting with the porous carbon material such as hydroxyl group, carboxyl group, amino group, etc., whereby molecular modification can also be achieved. Examples of the activating treatment include gas activating methods and chemical activating methods. Here, the gas activating method means a method in which oxygen, water vapor, carbon dioxide gas, air or the like is used as an activating agent and the porous carbon material is heated in such a gas atmosphere at 700 to 1000°C for a time ranging from several tens of minutes to several hours, whereby a fine structure is developed by the functions of volatile components or carbon molecules in the porous carbon material. Incidentally, the heating temperature may be appropriately selected based on the kind of raw material for the porous carbon material, the kind and/or concentration of the gas, etc., and is preferably 800 to 950°C. The chemical activating method means a method in which activation is conducted using zinc chloride, iron chloride, calcium phosphate, calcium hydroxide, magnesium carbonate, potassium carbonate, sulfuric acid or the like, in place of oxygen or water vapor used in the gas activating method, followed by washing with hydrochloric acid, pH adjustment with an alkaline aqueous solution, and drying.

In the drug sustained-release agents according to the first to third embodiment of the present invention inclusive of the above-mentioned preferable modes, preferably, a drug is adsorbed or supported on the porous carbon material in an amount of 1 to 200 parts by weight based on 100 parts by weight of the porous carbon material, whereby it is possible to obtain drug sustained-release agents which have carrier-drug composite bodies and in which the drug release rate is controlled appropriately. Here, examples of the drug include organic molecules, polymer molecules, and proteins. Specific examples of the drug include ibuprofen, pentoxifylline, prazosin, acyclovir, nifedipine, diltiazem, naproxen, flurbiprofen, ketoprofen, fenoprofen, indometacin, diclofenac, fentiazac, estradiol valerate, metoprolol, sulpiride, captopril, cimetidine, zidovudine, nicardipine, terfenadine, atenolol, salbutamol, carbamazepine, ranitidine, enalapril, simvastatin, fluoxetine, alprazolam, famotidine, ganciclovir, famciclovir, spironolactone, 5-asa, quinidine, perindopril, morphine, pentazocine, paracetamol, omeprazole, metoclopramide, aspirin, and metformin. From the viewpoint of systemic or local therapy, other examples of the drug include various hormones (e.g., insulin, estradiol, etc.), remedies for asthma (e.g., albuterol, etc.), remedies for tuberculosis (e.g., rifampicin, ethambutol, streptomycin, isoniazid, pyrazinamide, etc.), remedies for cancer (e.g., cisplatin, carboplatin, adriamycin, 5-FU, paclitaxel, etc.), remedies for hypertension (e.g., clonidine, prazosin, propranolol, labetalol, bunitrolol, reserpine, nifedipine, furosemide, etc.), which are non-limitative. Each of these drugs is dissolved in an organic solvent capable of dissolving the drug, the porous carbon material in the present invention is immersed in the solution, and then the solvent and surplus solute are removed, whereby it is possible to obtain a drug sustained-release agent having a porous carbon material-drug composite body. Specific examples of the solvent include water, methyl alcohol, ethyl alcohol, isopropyl alcohol, butyl alcohol, acetone, ethyl acetate, chloroform, 2-chloromethane, 1-chloromethane, hexane, tetrahydrofuran, and pyridine.

The adsorbents for adsorbing an organic matter thereon, or the adsorbents for medical use or the adsorbents for oral administration according to the first to third embodiments of the present invention can be used to selectively adsorbing thereon various unnecessary molecules present in a living body. Specifically, the adsorbents for adsorbing an organic matter thereon according to the first to third embodiments of the invention can be used as an adsorbent for medical use or an adsorbent for oral administration for internal medicine or the like effective for therapy or prevention of a disease. More specifically, in the case where the adsorbents for adsorbing an organic matter thereon according to the first to third embodiments of the invention are applied in the field of adsorbents for oral administration or adsorbents for medical use, or in the adsorbents for medical use or adsorbents for oral administration according to the first to third embodiments of the invention, examples of the organic matter to be adsorbed include indole, creatinine, uric acid, adenosine, Alizarine Cyanine Green, lysozyme, $\alpha$-amylase, albumin, 3-methylindole (skatole), tryptophan, indicant, theophylline, inosine 5-monophosphate disodium salt, and adenosine 5-triphosphate disodium salt. Besides, examples of the organic matter include organic matters (e.g., organic molecules, or proteins) having a number average molecular weight of $1 \times 10^2$ to $1 \times 10^5$, preferably $1 \times 10^2$ to $5 \times 10^4$, more preferably $1 \times 10^2$ to $2 \times 10^4$, further preferably $1 \times 10^2$ to $1 \times 10^4$. Further, examples of the organic matter include ammonia, urea, dimethylamine, guanidine compounds such as methylguanidine, etc., sulfur-containing amino acid, phenol, p-cresol, oxalic acid, homocysteine, guadininosuccinic acid, myo-inositol, indoxyl sulfate, pseudouridine, cyclic adenosine monophosphoric acid, $\beta$-aminoisobutyric acid, octopamine, $\alpha$-aminobutyric acid, parathyroid hormone, $\beta$2-microglobulin, ribonuclease, natriuretic hormone, water-soluble salts such as aspartic acid, arginine, etc. and amphoteric substances.

Examples of the organic matter further include purine and purine derivatives, adenine and guanine which are purine bases, guanosine and inosine which are purine nucleoside, adenylic acid, guanylic acid and inosinic acid which are purine nucleotides. Examples of the organic matter further include oligonucleotide and polynucleotide which are low-molecular or high-molecular nucleic acids. Examples of the organic matter further include polyamines, 3-deoxyglucosone, various peptide hormones, granulocyte inhibitory protein (GIP), degranulation inhibitory protein (DIP), and chemical migration inhibitory protein. Examples of the organic matter further include carbamoylated hemoglobin, saccharification end products, granulocytic/monocytic function inhibitor, oxidation accelerating agent, etc. Or, the adsorbent for adsorbing an organic matter thereon according to the first to third embodiments of the present invention can be used as a packing agent (absorbent) for hemocatharsis columns. Or, the adsorbents for adsorbing an organic matter thereon according to the first to third embodiments of the invention can be used as a water-cleaning adsorbent for cleaning of water.

In the adsorbents for adsorbing an allergen thereon according to the first to third embodiments of the present invention, examples of the allergen include allergens (Der p 1) arising from mites and an allergen (Cry j 1) arising from pollen of Japanese cedar, which are non-limitative. Here, an allergen means an antigen which reacts specifically with an antibody in a person having allergosis; generally, it means a substance which causes an allergic symptom or a substance which may well cause allergy. Incidentally, other examples of allergen include interior dust (so-called house dust such as polypide or feces of Dermatophagoides), squamae (dander of pets such as dogs and cats), pollens (pollen of Japanese green alder, pollens of Poaceae, pollens of Asteraceae, etc.), and fungi.

The functional food according to the first embodiment of the present invention for attaining the above object is a functional food having a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g, preferably not less than $1 \times 10^3$ m$^2$/g.

The functional food according to the second embodiment of the invention for attaining the above object is a functional food having a porous carbon material in which pores are arrayed in an arrangement corresponding to a crystal structure on a macroscopic basis, and the functional food according to the third embodiment of the invention for attaining the above object is a functional food having a porous carbon material in which pores are arrayed at a surface thereof in an arrangement corresponding to the (111) plane orientation of a face-centered cubic structure on a macroscopic basis. Incidentally, the functional foods according to the first embodiment, the second embodiment, and the third embodiment of the invention may contain other ingredients than the porous carbon material, for example, excipient, binder, disintegrator, lubricant, dilution agent flavoring agent, preservative, stabilizing agent, coloring matter, perfume, vitamins, color fixative, brightener, sweetening agent, bitterness agent, sour agent, taste enhancer, fermentative seasoning agent, antioxidant, enzyme, yeast extract, or nutritional supplement. Besides, examples of the form of the functional food include powdery form, solid form, tablets, particles, granules, capsules, creamy form, sol, gel, and colloid.

The masks according to the first embodiment, the second embodiment, and the third embodiment of the present invention for attaining the above object have the adsorbent for adsorbing an organic matter thereon according to the first embodiment of the invention, the adsorbent for adsorbing an organic matter thereon according to the second embodiment of the invention, and the adsorbent for adsorbing an organic matter thereon according to the third embodiment of the invention, respectively. Here, examples of the masks according to the first embodiment, the second embodiment, and the third embodiment of the invention include a mask for coping with pollinosis, in which protein, for example, is adsorbed by the adsorbent. Or, an allergen is adsorbed by the adsorbent.

The adsorption sheets according to the first embodiment, the second embodiment, and the third embodiment of the present invention for attaining the above object include a sheet-shaped member and a support member for supporting the sheet-shaped member, the sheet-shaped member having the adsorbent for adsorbing an organic matter thereon according to the first embodiment of the invention, the adsorbent for adsorbing an organic matter thereon according to the second embodiment of the invention, and the adsorbent for adsorbing an organic matter thereon according to the third embodiment, respectively.

The porous carbon materials constituting the drug sustained-release agents according to the first to third embodiments of the present invention, the porous carbon materials constituting the adsorbents for adsorbing an organic matter thereon according to the first to third embodiments of the invention, the porous carbon materials constituting the adsorbents for medical use according to the first to third embodiments of the invention, the porous carbon materials constituting the adsorbents for oral administration according to the first to third embodiments of the invention, the porous carbon materials constituting the adsorbents for adsorbing an allergen thereon according to the first to third embodiments of the invention, the porous carbon materials constituting the functional foods according to the first to third embodiments of the invention, the porous carbon materials constituting the marks according to the first to third embodiments of the invention, and the porous carbon materials constituting the adsorption sheets according to the first to third embodiments of the invention will sometimes be referred to generically as "the porous carbon material in the present invention." In addition, the porous carbon material constituting the drug sustained-release agent according to the first embodiment of the present invention, the porous carbon material constituting the adsorbent for adsorbing an organic matter thereon according to the first embodiment of the invention, the porous carbon material constituting the adsorbent for medical use according to the first embodiment of the invention, the porous carbon material constituting the adsorbent for oral administration according to the first embodiment of the invention, the porous carbon material constituting the adsorbent for adsorbing an allergen thereon according to the first embodiment of the invention, the porous carbon material constituting the functional food according to the first embodiment of the invention, the porous carbon material constituting the mask according to the first embodiment of the invention, and the porous carbon material constituting the adsorption sheet according to the first embodiment of the invention will sometimes be referred to generically as "the porous carbon material in the first embodiment of the present invention." Further, the porous carbon material constituting the drug sustained-release agent according to the second embodiment of the present invention, the porous carbon material constituting the adsorbent for adsorbing an organic matter thereon according to the second embodiment of the invention, the porous carbon material constituting the adsorbent for medical use according to the second embodiment of the invention, the porous carbon material constituting the adsorbent for oral administration according to the second embodiment of the invention, the porous carbon material constituting the adsorbent for adsorbing an allergen thereon according to the second embodiment of the invention, the porous carbon material constituting the functional food according to the second embodiment of the invention, the porous carbon material constituting the mask according to the second embodiment of the invention, and the porous carbon material constituting the adsorption sheet according to the second embodiment of the invention will sometimes be referred to generically as "the porous carbon material in the second embodiment of the present invention." Besides, the porous carbon material constituting the drug sustained-release agent according to the third embodiment of the present invention, the porous carbon material constituting the adsorbent for adsorbing an organic matter thereon according to the third embodiment of the invention, the porous carbon material constituting the adsorbent for medical use according to the third embodiment of the invention, the porous carbon material constituting the adsorbent for oral administration according to the third embodiment of the invention, the porous carbon material constituting the adsorbent for adsorbing an allergen thereon according to the third embodiment of the invention, the porous carbon material constituting the functional food according to the third embodiment of the invention, the porous carbon material constituting the mask according to the third embodiment of the invention, and the porous carbon material constituting the adsorption sheet according to the third embodiment of the invention will sometimes be referred to generically as "the porous carbon material in the third embodiment of the present invention."

In the porous carbon material in the present invention, pores forming the porous state have a three-dimensional regularity on nanoscale, specifically, the pores are arrayed in a three-dimensionally regular (ordered) manner, in other words, the porous carbon material has three-dimensional regularity and porosity on nanoscale. In this case, the porous carbon material can be manufactured, for example, based on the method disclosed in Japanese Patent Laid-open No. 2005-262324, as will be described later.

The porous carbon material in the present invention can be manufactured, for example, by impregnating inorganic particles on nanoscale (inorganic material particles or inorganic compound particles serving as a mold) with a monomer or a solution containing the monomer, polymerizing the monomer in this condition, then carbonizing the thus obtained polymer, and thereafter removing the inorganic particles. Here, the pores correspond to the cavities left after the removal of the inorganic particles. While the pores may be cavities partly closed in the carbon material insofar as they have a three-dimensional regularity, it is preferable that the pores each have a part piercing to (communicating with) the adjacent pore. In the porous carbon material in the present invention, different-sized pores may be contained, and, in this case, a pore arrangement pattern with a complicated regularity can also be obtained. In a method of manufacturing the porous carbon material which will be described later, the array of the pores is determined by the packing array of the inorganic particles, and, therefore, the arrayed state or arrayed structure of the inorganic particles is reflected on the regularity of the array of the pores.

In the porous carbon material in the first embodiment of the present invention, the pores have an average diameter $1 \times 10^{-9}$ to $1 \times 10^{-5}$ m, and the average diameter can be measured based on such a method as a mercury intrusion method, a nitrogen adsorption method, and SEM observation (provided that the size of the inorganic particles serving as a mold is measured by a light scattering method). In addition, the surface area of the porous carbon material in the first embodiment of the invention is not less than $3 \times 10^2$ m$^2$/g, and the surface area can be measured based on the BET method including adsorption of nitrogen; thus, the surface area is the so-called specific surface area.

In the porous carbon material in the second embodiment of the present invention, the array of pores is not particularly limited insofar as it is an arrangement corresponding to a crystal structure on a macroscopic basis. Examples of the crystal structure include face-centered cubic structure, body-centered cubic structure, and simple cubic structure. It is to be noted that, especially, the face-centered cubic structure, or the close-packed structure, is desirable from the viewpoint of increasing the surface area of the porous carbon material. The expression that the pores are arrayed in an arrangement corresponding to a crystal structure means that the pores are located at the arrangement positions of atoms in a crystal.

More preferably, the crystal structure is a single-crystal structure. Here, the expression "on a macroscopic basis" is used in the intension of excluding the case in which the arrangement (arranged state) corresponding to a crystal structure is observed only in a minute region (for example, a region with a size of 10 μm x 10 μm). For example, the expression means that the arranged state corresponding to a crystal structure is observed in a region with a size in excess of 10 μm × 10 μm. In addition, the expression means that the reflection spectrum to be described later shows absorption at substantially a single wavelength in any part of the porous carbon material and that the porous carbon material as a whole is monochromic. This applies also to the expression "on a macroscopic basis" in the porous carbon material in the third embodiment of the present invention.

In the porous carbon material in the third embodiment of the present invention, the pores are arrayed in an arranged state corresponding to the (111) plane orientation in a face-centered cubic structure. Specifically, this means that the pores are located at arrangement positions of atoms located on the (111) planes in the face-centered cubic structure.

In the porous carbon material in the second or third embodiment of the present invention, the shape of the pores is not particularly limited; for example, the shape of the pores is determined, to a certain extent, by the shape of the inorganic particles used in the manufacturing method thereof. Incidentally, taking the mechanical strength of the porous carbon material and the control of the shape of the inorganic particles on nanoscale into consideration, it is preferable that the shape of the pores is spherical (inclusive of a substantially spherical shape). Here, it is preferable that the average diameter of the pores is $1 \times 10^{-9}$ to $1 \times 10^{-5}$ m. If the average diameter of the pores is less than $1 \times 10^{-9}$ m (1 nm), the pores are too small and no conspicuous difference in characteristics can be seen between the porous carbon material and the bulk carbon material. On the other hand, if the average diameter of the pores exceeds $1 \times 10^{-5}$ m (10 $\mu$m), the pores are generally too large and the porous carbon material might be lowered in mechanical strength.

The method of manufacturing the porous carbon material in the present invention is fundamentally based on the method disclosed in Japanese Patent Laid-Open No. 2005-262324.

Specifically, the porous carbon material in the present invention can be manufactured by a manufacturing method which includes the steps of:

(A) immersing in a solution of a polymerizable monomer or a composition containing the polymerizable monomer a colloidal crystal body (which is a particulate material) insoluble in the solution, and then polymerizing the monomer to obtain a polymer; then

(B) carbonizing the polymer in an inert gas atmosphere at 800 to 3000°C; and thereafter

(C) immersing the colloidal crystal body in a solvent capable of dissolving the colloidal crystal body to dissolve and remove the colloidal crystal body.

Here, the colloidal crystal body means a crystal body in which colloidal particles are aggregated to be in an arranged state corresponding to a crystal structure and which has a three-dimensional regularity. In other words, the colloidal crystal body means a condition in which colloidal particles are located at the arrangement positions of atoms in a crystal.

Such a colloidal crystal body can be obtained by:

(1) a method in which a colloidal solution is dropped onto a substrate or into a flask and then the solvent is distilled off from the colloidal solution;

(2) a method in which a colloidal solution is subjected to suction filtration to remove the solvent; or

(3) a method in which a substrate is immersed in a colloidal solution, then the substrate is pulled up out of the colloidal solution, and the solvent is evaporated off from the colloidal solution. Or the colloidal crystal body can be obtained by a known method such as, for example:

(4) a method in which an electric field is applied to a colloidal solution and then the solvent is removed;

(5) a method in which two smooth substrates are arranged opposite to each other at an interval of several tens of micrometers in a colloidal solution having a comparatively low solid component concentration of 1 to 5 wt% in such a manner as to immerse lower portions of the substrates in the colloidal solution, thereby causing the colloidal solution to rise between the substrates by capillarity, and evaporating off and removing the solvent to deposit a colloidal crystal body between the two substrates;

(6) a method in which a colloidal solution in a dispersed state is left to stand, thereby depositing the colloidal particles by natural sedimentation, and then the solvent is removed; or

(7) a convective assembly method.

The porous carbon material obtained by use of a colloidal crystal body has a three-dimensional regularity and continuity in the array of pores on a macroscopic basis, and shows such an absorption characteristic that, when light is cast on the porous carbon material, there is little scattering of the reflectance of the reflected light, and that the reflection spectrum of the reflected light is unimodal.

In the method of (1) above, the distillation-off of the solvent can be performed at room temperature, but is preferably carried out by heating to a temperature of equal to or higher than the boiling point of the solvent used. Incidentally, the solvent may be distilled off by heating the substrate after the colloidal solution is dropped onto the substrate, or the solvent may be distilled off by dropping the colloidal solution onto a preheated substrate. During or after the dropping of the colloidal solution, the substrate may be rotated. By repeating an operation of dropping the colloidal solution and distilling off the solvent, or by regulating the concentration of the colloidal solution, or by regulating the amount of the colloidal solution dropped, or by appropriately combining these operations, it is possible to control the film thickness and/or area of the colloidal crystal body obtained. Especially, it is possible to enlarge the area of the colloidal crystal body while maintaining its three-dimensional regularity. Specifically, since a colloidal solution having a solid component concentration of not less than 10 wt% can be used, a colloidal crystal body having a considerable thickness can be formed on a substrate by dropping the colloidal solution only once. Film thickness can be controlled by repeating dropping and distilling (drying). Further, for example by using a monodisperse colloidal solution, it is possible to ensure that the colloidal crystal body obtained has a single-crystal structure.

As the method of (2) above, there can be specifically mentioned a method in which a colloidal solution containing colloidal particles is subjected to suction under reduced pressure by use of a suction funnel so as to remove the solvent under suction, thereby depositing a colloidal crystal body on a filter paper or filter fabric on the suction funnel. In this method, also, it is possible, for example by using a monodisperse colloidal solution, to ensure that the colloidal crystal body has a single-crystal structure. The concentration of the colloidal solution used in the suction filtration can be appropriately selected based on the volume of the colloidal crystal body to be obtained by one-time operation. Besides, a colloidal crystal body with a desired volume can be obtained also by once removing the solvent wholly under suction, thereafter adding the colloidal solution again, and repeating the same operation. By such a method of (2) above, also, it is possible to enlarge the area and/or volume of the colloidal crystal body while maintaining its three-dimensional regularity. Incidentally, the method for sucking the solvent is not particularly limited, and examples of the method include a method in which suction is conducted by an aspirator, a pump or the like. The speed of suction is also not particularly limited; specifically, a speed of constant lowering of the solution interface in a funnel at a vacuum degree of about 40 mmHg can be mentioned as a preferable example.

In the method of (3) above, also, it is possible, by regulating the concentration of the colloidal solution used and/or by repeating an operation, to obtain a colloidal crystal body having a desired area and/or a desired volume. The speed of pulling-up of the substrate is not particularly limited; since the colloidal crystal body grows at the interface between the colloidal solution and the atmospheric air, however, it is preferable to pull up the substrate at a slow speed. In addition, the speed (rate) of evaporation of the solvent also is not particularly limited, but a slow speed is preferable for the same reason as just-mentioned. It is possible, for example by using a monodisperse colloidal solution, to ensure that the colloidal crystal body obtained has a single-crystal structure. The status of the surface of the substrate to be used is not particularly restricted; it is preferable, however, to use a substrate whose surface is smooth.

The shape of the colloidal particles which are a particulate material used in the manufacture of the porous carbon material is preferably a true spherical shape or a roughly spherical shape. In addition, it is desirable, for example, to use inorganic particles soluble in fluorine compound solutions such as hydrofluoric acid, etc., alkaline solutions or acidic solutions. Specifically, examples of the inorganic material (inorganic compound) constituting the inorganic particles include carbonates, silicates, and phosphates of alkaline earth metals; metal oxides; metal hydroxides; other metal silicates; and other metal carbonates. More specifically, examples of the carbonates of alkaline earth metals include calcium carbonate, barium carbonate, and magnesium carbonate; examples of the silicates of alkaline earth metals include calcium silicate, barium silicate, and magnesium silicate; and examples of the phosphates of alkaline earth metals include calcium phosphate, barium phosphate, and magnesium phosphate.
In addition, examples of the metal oxides include silica, titanium oxide, iron oxide, cobalt oxide, zinc oxide, nickel oxide, manganese oxide, and aluminum oxide; and examples of the metal hydroxides include iron hydroxide, nickel hydroxide, aluminum hydroxide, calcium hydroxide, and chromium hydroxide. Further, examples of the other metal silicates include zinc silicate, and aluminum silicate; and examples of the other metal carbonates include zinc carbonate, and basic copper carbonate.
In addition, examples of natural matter include Shirasu-balloons, and pearlite.

The polymerizable monomer or composition containing the polymerizable monomer (specifically, a polymer capable of being converted into a porous carbon material) is not particularly restricted insofar as it is a polymer capable of being converted into a carbon material by carbonization. Specific examples of it include furfuryl alcohol resin, phenol-aldehyde resin, styrene-divinylbenzene copolymer, and furfuryl alcohol-phenol resin. It is more preferable to use a polymer such that a vitreous (amorphous) difficultly graphitizable carbon or easily graphitizable carbon or graphite (graphitized carbon) can be obtained as the porous carbon material by appropriately selecting the carbonizing temperature.

In order to obtain a polymer by polymerizing a monomer, before carbonization, in the presence of a particulate material such as the colloidal crystal body in which colloidal particles are arrayed in a regular manner on a three-dimensional basis, it is preferable that the concentration of the monomer is 0.1 to 99.9 wt% and the concentration of a crosslinking agent, which is added optionally, is 0.001 to 50 wt%. Besides, reaction conditions such as the concentration of a polymerization initiator, the polymerizing method, etc. may be appropriately selected from polymerization initiator concentration and reaction conditions which are suited to the monomer. For instance, a colloidal crystal body having colloidal particles arrayed in an ordered manner on a three-dimensional basis may be immersed in a solution obtained by dissolving a monomer, a catalyst, a polymerization initiator, a crosslinking agent and the like in a nitrogen-replaced organic solvent and thereafter the resulting body may be heated to an appropriate temperature or irradiated with energy rays such as light, whereby a polymer can be obtained through polymerization of the monomer. Specifically, based on a known method such as a radical polymerization method, a polycondensation method using an acid, etc. or on reversed-phase suspension polymerization or the like, a polymer can be obtained, for example, at a polymerization temperature of 0 to 100°C and in a polymerization time in the range from ten minutes to 48 hours.

In order to dissolve and remove the colloidal crystal body away from the composite body of the colloidal crystal body with the polymer, for example in the case where the colloidal crystal body is an inorganic material (inorganic compound), it suffices to use an acidic solution of a fluorine compound, an alkaline solution, an acidic solution or the like. For instance, in the case where the colloidal crystal body is formed of silica, Shirasu-balloons, or a silicate, it is required only to immerse the composite body in an aqueous hydrofluoric acid solution, or in an acidic solution of ammonium fluoride, calcium fluoride, sodium fluoride or the like, or in an alkaline solution of sodium hydroxide or the like. In the solution, it suffices that the amount of fluorine element is not less than four times the amount of silicon element in the composite body, and the concentration is preferably not less than 10 wt%. Besides, the alkaline solution is not specifically restricted insofar as it has a pH of not less than 11. In the case where the colloidal crystal body is a metal oxide or a metal hydroxide, it suffices to immerse the composite body in an acidic solution such as hydrochloric acid. The acidic solution is not particularly restricted insofar as it has a pH of not more than 3.

The dissolution and removal of the colloidal crystal body may be performed before or after the carbonization of the polymer obtained, and it suffices to carry out the dissolution and removal in an inert gas atmosphere at a temperature in the range of 800 to 3000°C. The temperature rise rate in raising the temperature to the carbonization temperature is not particularly limited insofar as it is in such a range that the structure of the polymer is not collapsed through localized heating.

Here, the carbonization generally means conversion of an organic material into a carbonaceous material by a heat treatment (see, for example, JIS M0104-1984). Incidentally, examples of an atmosphere for carbonization include an atmosphere shut off from oxygen, specifically, a vacuum atmosphere, an atmosphere of an inert gas such as nitrogen gas, argon gas, etc., and an atmosphere such as to steam and bake the organic material.

Advantageous Effects

In the drug sustained-release agents according to the first to third embodiments of the present invention, the adsorbents for adsorbing an organic matter thereon according to the first to third embodiments of the invention, the adsorbents for medical use according to the first to third embodiments of the invention, the adsorbents for adsorbing an allergen thereon according to the first to third embodiments of the invention, the adsorbents for oral administration according to the first to third embodiments of the invention, the functional foods according to the first to third embodiments of the invention, the masks according to the first to third embodiments of the invention, and the adsorption sheets according to the first to third embodiments of the invention, the average diameter and the arrayed state of the spherical pores in the porous carbon material are specified, or the arrayed state of the spherical pores in the porous carbon material is specified, so that it is possible to provide a porous carbon material having high performance and characteristics suitable for use in the drug sustained-release agents, the adsorbents for adsorbing an organic matter thereon, the adsorbents for medical use, the adsorbents for oral administration, the adsorbents for adsorbing an allergen thereon, the functional foods, the masks, or the adsorption sheets.

Brief Description of Drawings

[FIG. 1]

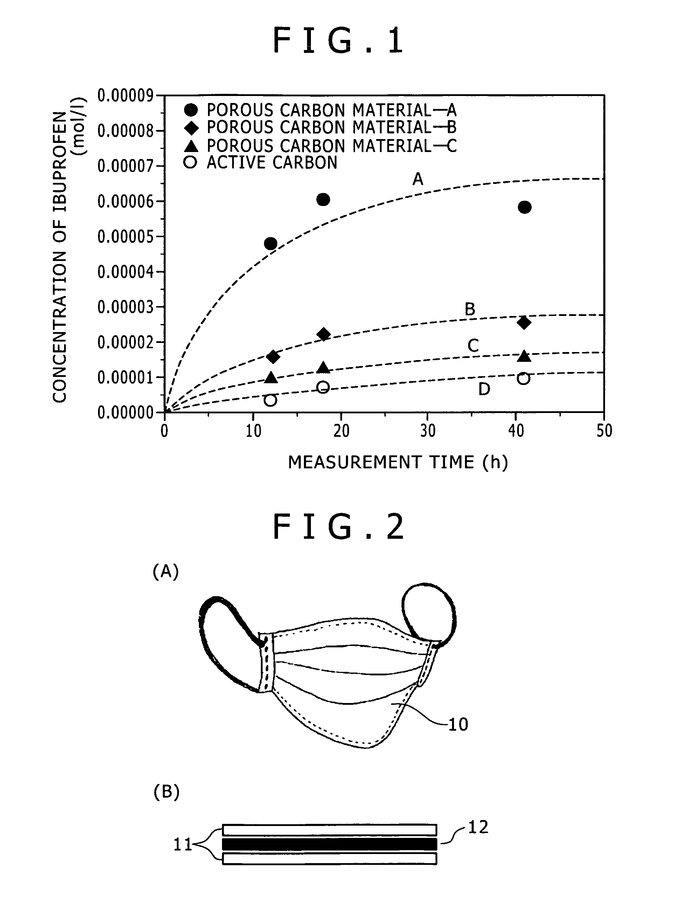

FIG. 1 is a graph showing measurement results of the concentration of ibuprofen at each time in Example 1 and Comparative Example 1.
[FIG. 2]
(A) and (B) of FIG. 2 are respectively a schematic illustration of a mask for coping with pollinosis in Example 4 and an illustration of a schematic sectional structure of a main body part of the mask.
[FIG. 3]

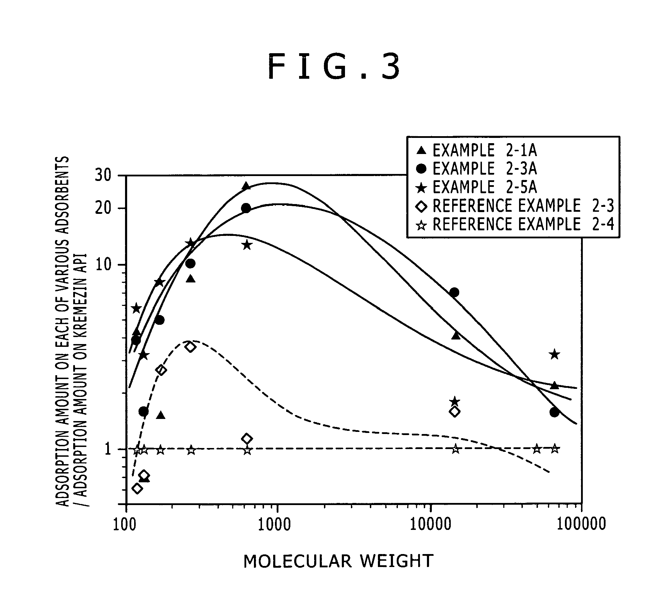

FIG. 3 is a graph showing normalized values of indole adsorption amount, creatinine adsorption amount, uric acid adsorption amount, adenosine adsorption amount, Alizarine Cyanine Green adsorption amount, lysozyme adsorption amount, and albumin adsorption amount, taking the adsorption amount per gram of Kremezin API as "1.0" in Reference Example 2-4.
[FIG. 4]

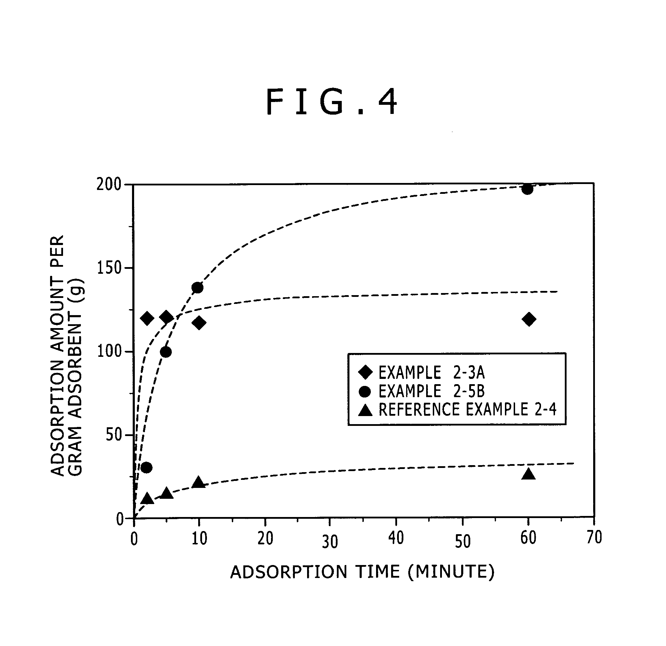

FIG. 4 is a graph showing examination results of the relationship between adsorption amount in an aqueous indole solution (aqueous solution A) and adsorption time, in Example 2-3A, Example 2-5B, and Reference Example 2-4.

Mode for Carrying Out the Invention

Now, the present invention will be described below based on Examples, referring to the drawings.

Example 1

Example 1 relates to the drug sustained-release agents according to the first to third embodiments of the present invention. In Example 1, first, porous carbon materials were manufactured based on the method described as follows.
Using monodisperse spherical particulates of silica (tradename: SEAHOSTAR KE) made by Nippon Shokubai Co., Ltd. or spherical particulates of silica (tradename: SNOWTEX) made by Nissan Chemical Industries, Ltd., a monodisperse aqueous silica colloidal suspension solution having an aqueous solution with a solid component concentration of 3 to 40 wt% was prepared. Incidentally, the colloidal particle diameter was in the range from 6 nm to 1 $\mu$m. The monodisperse aqueous silica colloidal suspension solution was poured into an SPC filter holder (made by Shibata Scientific Technology Ltd.) of 30 mm in diameter in which a filter fabric was placed, and suction under vacuum was conducted using an aspirator. Incidentally, the vacuum degree was set to about 40 mmHg. As a result, a silica colloidal layer could be obtained on the filter fabric. Incidentally, a polycarbonate membrane filter made by Whatman was used as the filter fabric. Then, after the filter fabric was peeled off, the filter cake was sintered in air at 1000°C for two hours, to obtain a thin film of colloidal crystal body (thin film-shaped silica colloidal single-crystal body).

Thereafter, the thin film-shaped silica colloidal single-crystal body was placed on a sheet of polytetrafluoroethylene, a mixture of 10.0 g of furfuryl alcohol and 0.05 g of oxalic acid hexahydrate (both made by Wako Pure Chemical Industries, Ltd.) was dropped onto the silica colloidal single-crystal body, and the surplus mixture flowing over from the thin film-shaped silica colloidal single-crystal body was lightly wiped away. Then, the silica colloidal single-crystal body with the mixture was placed in a desiccator, and evacuation was conducted several times to securely impregnate the crystal with the mixture. Thereafter, the crystal body impregnated with the mixture was held in air at 80°C for 48 hours, to effect polymerization.

Then, the silica-polymer resin composite body (polymer-colloidal crystal composite body) thus obtained was heated at 200°C in an argon atmosphere or a nitrogen gas atmosphere in a tubular furnace for one hour, for removal of water (moisture) and re-curing of the polymer resin. Next, temperature was raised at a rate of 5°C/min in an argon atmosphere, and carbonization was carried out at a constant temperature in the range of 800 to 1300°C for one hour, followed by cooling, to obtain a silica-carbon composite body (carbon material-colloidal crystal composite body).

Thereafter, the silica-carbon composite body was immersed in a 46% aqueous solution of hydrofluoric acid at room temperature for 24 hours, to dissolve the silica colloidal single-crystal body. Thereafter, washing with pure water and with ethyl alcohol was repeated until neutrality was reached, whereby a porous carbon material was obtained.

In the porous carbon material obtained as above, where the diameter of the colloidal particles was 50 nm, spherical pores with an average diameter of 50 nm were formed, with the adjacent pores interconnected via a through-hole of about 20 nm in size, and the surface area (specific surface area) of the porous carbon material was 1121 m$^2$/g. The porous carbon material produced using 280-nm silica particulates (SEAHOSTAR KEP30) was observed under a scanning electron microscope, whereon it was found that the pores were arrayed in an arrangement corresponding to a crystal structure on a macroscopic basis, or that the pores were arrayed at the surface of the porous carbon material in an arrangement corresponding to the (111) plane orientation of a face-centered cubic structure on a macroscopic basis. In addition, the porous carbon material was placed in a dark place, white light was cast on the porous carbon material at a glancing angle of 0°, and the wavelength of the reflected light was measured. The reflection spectrum thus obtained showed a unimodal absorption only in the vicinity of 450 nm. From this spectrum it was found that the pores were arrayed in a three-dimensionally regular (ordered) manner, also in the inside of the porous carbon material.

While varying the particle diameter of the monodisperse spherical particulates of silica, porous carbon materials were produced in the same manner as above-described. The average diameters of pores in the porous carbon materials thus obtained were 1 $\mu$m, 10 nm and 6 nm. Incidentally, for convenience, the porous carbon material with the average pore diameter of 1 $\mu$m will be referred to as "Porous Carbon Material A," while the porous carbon material with the average pore diameter of 10 nm will be referred to as "Porous Carbon Material B," and the porous carbon material with the average pore diameter of 6 nm will be referred to as "Porous Carbon Material C." The surface areas (specific surface areas) of the porous carbon material in Porous Carbon Material A, Porous Carbon Material B, and Porous Carbon Material C were as set forth below.

Porous Carbon Material A: 320 m$^2$/g
Porous Carbon Material B: 1488 m$^2$/g
Porous Carbon Material C: 1493 m$^2$/g

Each of the thus obtained Porous Carbon Material A, Porous Carbon Material B, and Porous Carbon Material C in an amount of 0.01 g was impregnated overnight with a solution prepared by dissolving 0.10 g of ibuprofen (non-steroidal antiinflammatory drug, NSAID) in 10 mL of hexane, and then filtration by use of a membrane filter was conducted, followed by vacuum drying at 40°C.

As Comparative Example 1, 0.01 g of a commercial active carbon (made by Wako Pure Chemical Industries, Ltd.) was immersed overnight with a solution prepared by dissolving 0.10 g of ibuprofen in 10 mL of hexane, and filtration by use of a membrane filter was conducted, followed by vacuum drying at 40°C.

Each of Drug Sustained-Release Agent A, Drug Sustained-Release Agent B, and Drug Sustained-Release Agent C of Example 1 composed of various porous carbon material-ibuprofen combinations and the drug sustained-release agent of Comparative Example 1 composed of an active carbon-ibuprofen combination was mixed with 40 mL of aqueous phosphate buffer solution (pH 7.3), and the concentration of ibuprofen at each time was measured and calculated, through ultraviolet spectroscopy. The measurement results for Drug Sustained-Release Agent A, Drug Sustained-Release Agent B, and Drug Sustained-Release Agent C of Example 1 and for the drug sustained-release agent composed of the active carbon-ibuprofen combination of Comparative Example 1 are shown in FIG. 1. Incidentally, in FIG. 1, the measurement result for Drug Sustained-Release Agent A composed of the Porous Carbon Material A-ibuprofen combination is denoted by "A." In addition, the measurement result for Drug Sustained-Release Agent B composed of the Porous Carbon Material B-ibuprofen combination is denoted by "B." Further, the measurement result for Drug Sustained-Release Agent C composed of the Porous Carbon Material C-ibuprofen combination is denoted by "C." Besides, the measurement result for the drug sustained-release agent composed of the active carbon-ibuprofen combination of Comparative Example 1 is denoted by "D." As seen from FIG. 1, in the drug sustained-release agents composed of the porous carbon material-ibuprofen combinations of Example 1, the value of sustained release quantity was greater as the average diameter of the pores in the porous carbon material was larger. Besides, the drug sustained-release agents of Example 1 showed much greater sustained release quantities as compared with that of Comparative Example 1.

Example 2

Example 2 relates to adsorbents for adsorbing an organic matter thereon according to the first to third embodiments of the present invention, adsorbents for medial use according to the first to third embodiments of the invention, and adsorbents for oral administration according to the first to third embodiments of the invention. In Example 2, Porous Carbon Material D, Porous Carbon Material E, Porous Carbon Material F, Porous Carbon Material G and Porous Carbon Material H were produced based on the same method as described in Example 1. Incidentally, Porous Carbon Material D, Porous Carbon Material E and Porous Carbon Material F were subjected to a nitric acid treatment as a chemical treatment, whereas Porous Carbon Material F and Porous Carbon Material G were subjected to an activating treatment by use of water vapor. The carbonizing temperature, the presence/absence of the nitric acid treatment, and the presence/absence of the activating treatment are set forth in Table 1. Then, for various substances, adsorption amount per unit weight of the porous carbon material was measured. Incidentally, the nitric acid treatment was carried out by adopting a method in which 3 g of a sample is added to 200 mL of concentrated nitric acid, the mixture is stirred for 12 hours, then filtration by use of a membrane filter is conducted, and washing with pure water is carried out, followed by drying. Besides, the water vapor activation was carried out by adopting a method in which water vapor is caused to flow through a burning furnace provided with a nitrogen atmosphere and set at 900°C, at a flow rate of 1 L/min for three hours.
In carrying out measurement of adsorption amount, first, aqueous solutions (Aqueous Solution A, Aqueous Solution B, Aqueous Solution C, Aqueous Solution D, Aqueous Solution E, Aqueous Solution F, Aqueous Solution G, Aqueous Solution H, Aqueous Solution I, Aqueous Solution J, Aqueous Solution K, Aqueous Solution L, Aqueous Solution M, Aqueous Solution N) having concentrations as shown in Table 4 below were prepared by use of 14 kinds of substances differing in number average molecular weight, namely, indole (number average M.W.: 117), creatinine (number average M.W.: 131), uric acid (number average M.W.: 168), adenosine (number average M.W.: 267), Alizarine Cyanine Green (number average M.W.: 623), lysozyme (number average M.W.: 14307), α-amylase (number average M.W.: about 50000), albumin (number average M.W.: about 66000), 3-methylindole (number average M.W.: 131), theophylline (number average M.W.: 180), L-tryptophan (number average M.W.: 204), indicant (number average M.W.: 295), inosine 5-monophosphate disodium salt (number average M.W.: 392), and adenosine 5-triphosphate disodium salt (number average M.W.: 551), together with a phosphate buffer of pH 7.3. Incidentally, the concentration of each aqueous solution before adsorption was determined arbitrarily. Then, 0.010 g of the porous carbon material was added to 40.0 mL of each of the aqueous solutions thus prepared, and the resulting admixture was shaken at $37\pm2$°C for one hour. After the shaking, the porous carbon material was removed from the aqueous solution by use of a polytetrafluoroethylene-made membrane filter having micropores of 500 $\mu$m. Then, absorbance of the filtrate was measured by UV-visible absorbance measurement, and the molar concentration of the aqueous solution was determined. Incidentally, the adsorption amount was calculated by comparing the thus determined molar concentration of the aqueous solution with an initial value of the molar concentration of the aqueous solution. The adsorption amount per gram of the porous carbon material was calculated based on the formula given below. Incidentally, indole, 3-methylindole, L-tryptophan, and indicant are toxins relevant to renal disease, and creatinine is a toxin relevant to renal disease and hepatic disease. In addition, uric acid is a toxin relevant to renal disease, and is a substance which may cause gout, arteriosclerosis or uratic calculus through hyperuricemia. Further, adenosine, inosine 5-monophosphate disodium salt, adenosine 5-triphosphate disodium salt correspond to purine bodies and analogues thereof. In addition, theophylline and Alizarine Cyanine Green are models of drug poisoning (provided that theophylline is a remedy for respiratory system disease, and Alizarine Cyanine Green is a synthetic coloring matter for foods), whereas lysozyme, α-amylase, and albumin are models for examining adsorption characteristics for such proteins as inflammatory cytokine which would cause Crohn's disease or the like (simulated inflammatory cytokine).

$$\text{(Adsorption amount per gram of porous carbon material)} =$$
$$\text{(Molecular weight of solute)} \times \{\text{(Molar concentration of aqueous}$$
$$\text{solution before adsorption)} - \text{(Molar concentration of aqueous}$$
$$\text{solution after adsorption)}\} / \text{(Amount of porous carbon material}$$
$$\text{per 1000 mL)}$$

Besides, for reference, as Reference Example 2-1, Reference Example 2-2, Reference Example 2-3 and Reference Example 2-4, adsorption amount per gram of active carbon was measured for active carbons set forth in Table 2 below. Incidentally, the porous carbon materials, measurement results of surface area (specific surface area) of active carbon, and measurement results of pore volume, in Example 2 and Reference Example 2 are set forth in Table 3.

[Table 1]

| Sample | | Carbonizing Temperature | Nitric acid treatment | Water vapor activating treatment |
|---|---|---|---|---|
| Ex.2 -1A | Porous carbon material D (pore: 50 nm) | 1000°C | absent | - - - |
| Ex.2 -1B | | 800°C | absent | - - - |
| Ex.2 -1C | | 1000°C | present | - - - |
| Ex.2 -2A | Porous carbon material E (pore: 1 0 nm) | 800°C | absent | - - - |
| Ex.2 -2B | | | present | - - - |
| Ex.2 - 3A | Porous carbon material F (pore: 6 nm) | 800°C | absent | - - - |
| Ex.2 - 3B | | | present | - - - |
| Ex.2 - 4A | Porous carbon material G (pore: 100 nm) | 800°C | - - - | absent |
| Ex.2 - 4B | | | - - - | present |
| Ex.2 - 5A | Porous carbon material H (pore: 1 _m) | 800°C | - - - | absent |
| Ex.2 - 5B | | | - - - | present |

[Table 2]

| | Name of product | Manufacturer | Raw material |
|---|---|---|---|
| Ref.Ex. 2-1 | Active carbon | Wako Pure Chemical Industries, Ltd. | Petroleum pitch |
| Ref.Ex. 2-2 | Active carbon (Kuraray Coal YP -17D) | Kuraray Chemical Co., Ltd. | Coconut shell |
| Ref.Ex. 2-3 | Active carbon (Kuraray Coal GW) | Kuraray Chemical Co., Ltd. | Coconut shell |
| Ref.Ex. 2-4 | Kremezin API | Kureha Corp. | Petroleum pitch |

[Table 3]

| | Specific surface area ($m^2/g$) | Pore volume ($cm^3/g$) |
|---|---|---|
| Example 2-1A | 1020 | 2.22 |
| Example 2-1B | 1122 | 2.61 |
| Example 2-1C | 1034 | 4.12 |
| Example 2-2A | 1488 | 2.49 |

(continued)

| | Specific surface area (m$^2$/g) | Pore volume (cm$^3$/g) |
|---|---|---|
| Example 2-2B | 1501 | 2.65 |
| Example 2-3A | 1493 | 2.20 |
| Example 2-3B | 1515 | 2.51 |
| Example 2-4A | 873 | 0.49 |
| Example 2-4B | 1387 | 0.82 |
| Example 2-5A | 320 | 0.45 |
| Example 2-5B | 1265 | 0.99 |
| Ref.Ex. 2-1 | 1231 | 0.57 |
| Ref.Ex. 2-2 | 1584 | 0.79 |
| Ref.Ex. 2-3 | 885 | 0.40 |
| Ref.Ex. 2-4 | 1079 | 0.60 |

[Table 4]

| | Solute | Molecular weight of solute | Molar concentration (mol/L) |
|---|---|---|---|
| Aq.Soln.A | Indole | 117 | $4.234\_10^{-4}$ |
| Aq.Soln.B | Creatinine | 131 | $3.567\_10^{-4}$ |
| Aq.Soln.C | Uric acid | 168 | $4.616\_10^{-4}$ |
| Aq.Soln.D | Adenosine | 267 | $1.669\_10^{-4}$ |
| Aq.Soln.E | Alizarine Cyanine Green | 623 | $2.416\_10^{-4}$ |
| Aq.Soln.F | Lysozyme | 14307 | $8.370\_10^{-5}$ |
| Aq.Soln.G | -Amylase | 50000 | $6.693\_10^{-6}$ |
| Aq.Soln.H | Albumin | 66000 | $6.533\_10^{-5}$ |
| Aq.Soln.I | 3-Methylindole | 131 | $4.574\_10^{-4}$ |
| Aq.Soln.J | Theophylline | 180 | $2.959\_10^{-4}$ |
| Aq.Soln.R | L-Tryptophan | 204 | $7.609\_10^{-4}$ |
| Aq.Soln.L | Indican | 295 | $6.103\_10^{-4}$ |
| Aq.Soln.M | Inosine 5 -monophospha te disodium s alt | 392 | $4.136\_10^{-4}$ |
| Aq.Soln.N | Adenosine 5 -triphosphate disodium salt | 551 | $2.141\_10^{-4}$ |

Indole adsorption amount (mg), creatinine adsorption amount (mg), uric acid adsorption amount (mg), adenosine adsorption amount (mg), Alizarine Cyanine Green adsorption amount (mg), lysozyme adsorption amount (mg), $\alpha$-amylase adsorption amount (mg), albumin adsorption amount (mg), 3-methylindole adsorption amount (mg), theophylline adsorption amount (mg), L-tryptophan adsorption amount (mg), indicant adsorption amount (mg), inosine 5-monophosphate disodium salt adsorption amount (mg), and adenosine 5-triphosphate disodium salt adsorption amount (mg), per gram of porous carbon material in Example 2 or of active carbon in Reference Example are shown in Table 5, Table 6, Table 7, Table 8, Table 9, Table 10, Table 11, Table 12, Table 13, Table 14, Table 15, Table 16, Table 17, and Table 18 below. As shown in Tables 5 to 18, the adsorption amounts on the samples of Examples 2-1A to 2-5B were found to be greater than the adsorption amounts on the samples of Reference Examples 2-1 to 2-4. Besides, normalized values of indole adsorption amount, creatinine adsorption amount, uric acid adsorption amount, adenosine adsorption amount, Alizarine Cyanine Green adsorption amount, lysozyme adsorption amount, and $\alpha$-amylase adsorption amount, determined based on the results shown in Tables 5 to 18 and taking the adsorption amount per gram of Kremezin API of Reference Example 2-4 as "1.0" are shown in Table 19. Similarly, normalized values of 3-methyl indole adsorption amount, theophylline adsorption amount, L-tryptophan adsorption amount, indicant adsorption amount, inosine 5-monophosphate disodium salt adsorption amount, and adenosine 5-triphosphate disodium salt adsorption amount determined in the same method are shown in Table 20. Besides, a graph showing the normalized values of indole adsorption amount, creatinine adsorption amount, uric acid adsorption amount, adenosine adsorption amount, Alizarine Cyanine Green adsorption amount, lysozyme adsorption amount, and albumin adsorption amount, determined taking the adsorption amount per gram of Kremezin API of Reference Example 2-4 as "1.0" is shown in FIG. 3. Further, a graph showing examination results of the relation between adsorption amount, in an aqueous indole solution (Aqueous Solution A) in Example 2-3A, Example 2-5B, and Reference Example 2-4, and adsorption time is shown in FIG. 4. As seen from Table 19, Table 20 and FIG. 3, also, the adsorbents in Example 2 effectively adsorb thereon organic matters having a number average molecular weight in the range of $1 \times 10^2$ to $5 \times 10^4$, preferably $1 \times 10^2$ to $2 \times 10^4$, more preferably $1 \times 10^2$ to $1 \times 10^4$.

[Table 5]

| Indole adsorption amount (mg) per gram of porous carbon material or the like | |
|---|---|
| Molecular weight of indole: 117 | |
| | Indole adsorption amount (mg) |
| Example 2-1B | 141.73 |
| Example 2-1C | 124.04 |
| Example 2-2A | 140.40 |
| Example 2-2B | 95.64 |
| Example 2-3A | 127.90 |
| Example 2-3B | 97.02 |
| Example 2-5A | 91.65 |
| Example 2-5B | 192.05 |
| Ref.Ex. 2-3 | 24.10 |
| Ref.Ex. 2-4 | 32.88 |

[Table 6]

| Creatinine adsorption amount (mg) per gram of porous carbon material or the like Molecular weight of creatinine: 131 | |
|---|---|
| | Creatinine adsorption amount (mg) |
| Example 2-1A | 24.67 |
| Example 2-2A | 24.03 |
| Example 2-2B | 23.69 |
| Example 2-3A | 26.85 |
| Example 2-3B | 23.66 |
| Example 2-4B | 40.52 |
| Example 2-5B | 53.84 |
| Ref.Ex. 2-3 | 12.18 |
| Ref.Ex. 2-4 | 16.88 |

[Table 7]

| Uric acid adsorption amount (mg) per gram of porous carbon material or the like | |
|---|---|
| Molecular weight of uric acid: 168 | |
| | Uric acid adsorption amount (mg) |
| Example 2-1C | 73.89 |
| Example 2-2A | 71.27 |
| Example 2-2B | 40.28 |
| Example 2-3A | 69.51 |
| Example 2-3B | 42.42 |
| Example 2-5B | 112.15 |
| Ref.Ex. 2-3 | 36.94 |
| Ref.Ex. 2-4 | 13.86 |

[Table 8]

| Adenosine adsorption amount (mg) per gram of porous carbon material or the like Molecular weight of adenosine: 267 | |
|---|---|
| | Adenosine adsorption amount (mg) |
| Example 2-1B | 112.28 |
| Example 2-1C | 125.29 |
| Example 2-2A | 145.51 |
| Example 2-2B | 126.98 |
| Example 2-3A | 136.69 |
| Example 2-3B | 113.88 |
| Example 2-5B | 178.23 |
| Ref.Ex. 2-3 | 48.07 |
| Ref.Ex. 2-4 | 13.45 |

[Table 9]

| Alizarine Cyanine Green adsorption amount (mg) per gram of porous carbon material or the like Molecular weight of Alizarine Cyanine Green: 623 | |
|---|---|
| | Alizarine Cyanine Green adsorption amount (mg) |
| Example 2-1A | 491.86 |
| Example 2-1B | 539.26 |
| Example 2-1C | 389.47 |
| Example 2-2A | 404.10 |
| Example 2-2B | 309.47 |
| Example 2-3A | 408.10 |
| Example 2-3B | 320.68 |
| Example 2-4A | 355.05 |

(continued)

| Alizarine Cyanine Green adsorption amount (mg) per gram of porous carbon material or the like Molecular weight of Alizarine Cyanine Green: 623 | |
|---|---|
| | Alizarine Cyanine Green adsorption amount (mg) |
| Example 2-4B | 591.45 |
| Example 2-5B | 278.24 |
| Ref.Ex. 2-1 | 82.82 |
| Ref.Ex. 2-2 | 213.25 |
| Ref.Ex. 2-3 | 4.54 |
| Ref.Ex. 2-4 | 20.53 |

[Table 10]

| Lysozyme adsorption amount (mg) per gram of porous carbon material or the like Molecular weight of lysozyme: 14307 | |
|---|---|
| | Lysozyme adsorption amount (mg) |
| Example 2-1A | 243.61 |
| Example 2-1C | 898.49 |
| Example 2-2A | 413.82 |
| Example 2-2B | 253.84 |
| Example 2-3A | 315.18 |
| Example 2-3B | 252.42 |
| Example 2-5A | 136.41 |
| Ref.Ex. 2-1 | 84.02 |
| Ref.Ex. 2-2 | 109.54 |
| Ref.Ex. 2-3 | 91.66 |
| Ref.Ex. 2-4 | 58.24 |

[Table 11]

| $\alpha$-Amylase adsorption amount (mg) per gram of porous carbon material or the like | |
|---|---|
| Molecular weight of $\alpha$-amylase: 50000 | |
| | - Amylase adsorption amount (mg) |
| Example 2-3A | 77.74 |
| Example 2-5B | 128.20 |
| Ref.Ex. 2-4 | 37.84 |

[Table 12]

| Albumin adsorption amount (mg) per gram of porous carbon material or the like Molecular weight of albumin: 66000 | |
|---|---|
| | Albumin adsorption amount (mg) |
| Example 2-1A | 573.22 |
| Example 2-1C | 1111.96 |
| Example 2-2A | 571.44 |
| Example 2-2B | 362.43 |
| Example 2-3A | 450.08 |
| Example 2-3B | 370.74 |
| Example 2-4A | 1003.76 |
| Example 2-4B | 1358.41 |
| Example 2-5A | 824.61 |
| Example 2-5B | 1302.51 |
| Ref.Ex. 2-2 | 181.71 |
| Ref.Ex. 2-3 | 1.39 |

[Table 13]

| 3-Methylindole adsorption amount (mg) per gram of porous carbon material or the like Molecular weight of 3-methylindole: 131 | |
|---|---|
| | 3-Methylindole adsorption amount (mg) |
| Example 2-1B | 163.61 |
| Example 2-1C | 156.33 |
| Example 2-2A | 183.71 |
| Example 2-3A | 160.68 |
| Example 2-5B | 228.69 |
| Ref.Ex. 2-4 | 117.08 |

[Table 14]

| Theophylline adsorption amount (mg) per gram of porous carbon material or the like Molecular weight of theophylline: 180 | |
|---|---|
| | Theophylline adsorption amount (mg) |
| Example 2-1B | 149.66 |
| Example 2-2A | 177.42 |
| Example 2-3A | 171.14 |
| Example 2-5B | 202.43 |
| Ref.Ex. 2-3 | 48.58 |
| Ref.Ex. 2-4 | 38.39 |

[Table 15]

| L-Tryptophan adsorption amount (mg) per gram of porous carbon material or the like  Molecular weight of L-tryptophan: 204 | |
|---|---|
| | L-Tryptophan adsorption amount (mg) |
| Example 2-2A | 192.62 |
| Example 2-3A | 188.54 |
| Example 2-5B | 248.89 |
| Ref.Ex. 2-3 | 68.28 |
| Ref.Ex. 2-4 | 111.63 |

[Table 16]

| Indican adsorption amount (mg) per gram of porous carbon material or the like  Molecular weight of indican: 295 | |
|---|---|
| | Indican adsorption amount (mg) |
| Example 2-1B | 331.06 |
| Example 2-2A | 274.16 |
| Example 2-5B | 339.71 |
| Ref.Ex. 2-3 | 46.93 |
| Ref.Ex. 2-4 | 148.50 |

[Table 17]

| Inosine 5-monophosphate disodium salt adsorption amount (mg) per gram of porous carbon material or the like  Molecular weight of inosine 5-monophosphate  disodium salt: 392 | |
|---|---|
| | Inosine 5-monophosphate disodium salt adsorption amount (mg) |
| Example 2-2A | 190.48 |
| Example 2-3A | 194.32 |
| Example 2-5B | 235.25 |
| Ref.Ex. 2-3 | 56.46 |
| Ref.Ex. 2-4 | 68.89 |

[Table 18]

| Adenosine 5-triphosphate disodium salt adsorption amount (mg) per gram of porous carbon material or the like  Molecular weight of adenosine 5-triphosphate disodium salt: 551 | |
|---|---|
| | Adenosine 5-triphosphate disodium salt adsorption amount (mg) |
| Example 2-2A | 115.46 |
| Example 2-3A | 128.54 |
| Example 2-5B | 85.73 |
| Ref.Ex. 2-3 | 8.54 |

(continued)

| Adenosine 5-triphosphate disodium salt adsorption amount (mg) per gram of porous carbon material or the like<br>Molecular weight of adenosine 5-triphosphate disodium salt: 551 | |
|---|---|
| | Adenosine 5-triphosphate disodium salt adsorption amount (mg) |
| Ref.Ex. 2-4 | 47.79 |

[Table 19]

| | Indole A.A. | Creatinine A.A. | Uric Acid A.A. | Adenosine A.A. | Alizarine Cyanine Green A.A | Lysozyme A.A. | -Amylase A.A. |
|---|---|---|---|---|---|---|---|
| Number average MW | 117 | 131 | 168 | 267 | 623 | 14307 | 50000 |
| Ex. 2-1A | | 1.5 | | | 24.0 | 4.2 | |
| Ex. 2-1B | 4.3 | | | 8.3 | 26.3 | | |
| Ex. 2-1C | 3.8 | | 5.3 | 9.3 | 19.0 | 15.4 | |
| Ex. 2-2A | 4.3 | 1.4 | 5.1 | 10.8 | 19.7 | 7.1 | |
| Ex. 2-2B | 2.9 | 1.4 | 2.9 | 9.4 | 15.1 | 4.4 | |
| Ex. 2-3A | 3.9 | 1.6 | 5.0 | 10.2 | 19.9 | 5.4 | 2.1 |
| Ex. 2-3B | 3.0 | 1.4 | 3.1 | 8.5 | 15.6 | 4.3 | |
| Ex. 2-4A | | | | | 17.3 | | |
| Ex. 2-4B | | 2.4 | | | 28.8 | | |
| Ex. 2-5A | 2.8 | | | | | 2.3 | |
| Ex. 2-5B | 5.8 | 3.2 | 8.1 | 13.3 | 13.6 | | 3.4 |
| A.A.: adsorption amount | | | | | | | |

[Table 20]

| | 3-Methyl indole | Theophyline | L-Tryptophan | Indican | Inosine 5-mono -phosphate disodium salt | Adenosine 5-tri -phosphate disodium salt |
|---|---|---|---|---|---|---|
| Number average MW | 131 | 180 | 204 | 295 | 392 | 551 |
| Ex. 2 -1B | 1.4 | 3.9 | | 2.2 | | |
| Ex. 2 -1C | 1.3 | | | | | |
| Ex. 2 -2A | 1.6 | 4.6 | 1.7 | 1.8 | 2.8 | 2.4 |
| Ex. 2 -3A | 1.4 | 4.5 | 1.7 | | 2.8 | 2.7 |
| Ex. 2 -sub | 2.0 | 5.3 | 2.2 | 2.3 | 3.4 | 1.8 |

Example 3

Example 3 relates to the adsorbents for adsorbing an allergen thereon according to the first to third embodiments of the present invention. In Example 3, the sample of Example 2-3B (see Table 1) was used as the porous carbon material. Besides, as a reference example, the sample of Reference Example 2-1 (see Table 2) was used. For the samples of Example 2-3B and Reference Example 2-1, the adsorption amounts of an allergen (Der p 1) arising from mites and an allergen (Cry j 1) arising from the pollen of Japanese cedar, per gram of the sample, were measured. Measurement of adsorption amount was fundamentally carried out by the same method as in Example 2. The molecular weight of Der p 1 is 36000 to 40000, and the molecular weight of Cry j 1 is 50000. The adsorption amounts on the sample of Example 2-3B, with the adsorption amount on the sample of Reference Example 2-1 being taken as "1," were as set forth in Table 21 below. Thus, it is seen that the adsorbent in Example 3 adsorbs the allergens thereon effectively.

[Table 21]

| | |
|---|---|
| Der p 1 | 2.94 times |
| Cry j 1 | 1.50 times |

Example 4

Example 4 relates to the masks and the adsorption sheets according to the first to third embodiments of the present invention. A schematic illustration of a mask for coping with pollinosis is shown in FIG. 2 (A), and a schematic sectional view of a main body part (adsorption sheet) of the mask for coping with pollinosis is shown in FIG. 2 (B). The main body part 10 of the mask for coping with pollinosis has a structure in which a sheet-shaped porous carbon material 12 is sandwiched between non-woven cellulose fabrics 11 and 11.

In order to form a porous carbon material described in Example 1 into a sheet-like shape, a method may be adopted in which, for example, a porous carbon material-polymer composite body is formed by use of carboxynitrocellulose as a binder. On the other hand, the adsorption sheet of Example 4 includes a sheet-shaped member composed of a porous carbon material described in Example 1 (specifically, a porous carbon material-polymer composite body using carboxynitrocellulose as a binder), and a support member for supporting the sheet-shaped member (specifically, non-woven fabrics as support members between which the sheet-shaped member is sandwiched). It is considered that when the porous carbon material in the present invention is applied to an adsorbent in various masks such as a mask for coping with pollinosis, for example, a protein portion of pollen is adsorbed on the porous carbon material, whereby the pollen can be effectively adsorbed. Or, the allergen can be effectively adsorbed.

Example 5

Example 5 is a modification of Examples 1 to 4. In Example 5, a monodisperse aqueous silica colloidal suspension solution composed of an aqueous solution with a solid component concentration of 15% was prepared by use of the monodisperse spherical particulates of silica described in Example 1. Then, the monodisperse aqueous silica colloidal suspension solution was poured into a vessel or beaker made of polytetrafluoroethylene and having a volume of 3 cm × 3 cm × 0.5 cm, and the solvent was evaporated off at 100°C, to obtain a colloidal crystal body composed of silica particulates.

Thereafter, a mixture of 10.0 g of furfuryl alcohol and 0.05 g of oxalic acid hexahydrate (both made by Wako Pure Chemical Industries, Ltd.) was poured to the colloidal crystal body in the vessel, and the surplus mixture flowing over from the colloidal crystal body was lightly wiped away. Then, the colloidal crystal body with the mixture was placed in a desiccator, and evacuation was carried out several times, to securely impregnate the crystal with the mixture. Thereafter, polymerization was carried out in air at 80°C for 48 hours.

Then, the silica-polymer resin composite body (polymer-colloidal crystal composite body) thus obtained was taken out of the vessel, and was heated in an argon atmosphere in a tubular furnace at 200°C for one hour, for removal of water (moisture) and re-curing of the polymer resin. Next, in an argon atmosphere, temperature was raised at a rate of 5°C/min to 1000°C, and carbonization was effected at 1000°C for 1 hour, followed by natural cooling, to obtain a silica-carbon composite body (carbon material-colloidal crystal composite body).

Thereafter, the composite body was immersed in an aqueous 46% solution of hydrofluoric acid at room temperature for 24 hours, to dissolve the silica colloidal single-crystal body. Thereafter, washing with pure water and ethyl alcohol was repeated until neutrality was reached, to obtain a porous carbon material.

The porous carbon material thus obtained was also found to have substantially the same structure as that of the porous carbon material obtained in Example 1.

Then, based on the porous carbon material thus obtained, a drug sustained-release agent similar to that in Example 1 was obtained. The drug sustained-release agent thus obtained showed characteristics similar to those of the drug sustained-release agent in Example 1. In addition, based on the porous carbon material obtained above, an adsorbent for adsorbing an organic matter thereon similar to that in Example 2 was obtained. The adsorbent for adsorbing an organic matter thereon thus obtained showed characteristics similar to those of the adsorbent for adsorbing an organic matter thereon in Example 2. Besides, the adsorbent obtained in this example showed characteristics similar to those of the adsorbent for adsorbing an allergen thereon in Example 3. Furthermore, based on the porous carbon material obtained in this example, a mask and an adsorption sheet similar to those in Example 4 could be obtained.

Example 6

Example 6 also is a modification of Examples 1 to 4. In Example 6, a porous carbon material was obtained by the same process as in Example 1, except that after a silica-polymer resin composite body (polymer-colloidal crystal composite body) was obtained, the silica colloidal single-crystal body was dissolved by use of a hydrofluoric acid solution and, thereafter, carbonization was conducted. The porous carbon material thus obtained was also found to have substantially the same structure as that of the porous carbon material obtained in Example 1.

Then, based on the porous carbon material thus obtained, a drug sustained-release agent similar to that in Example 1 was obtained. The drug sustained-release agent thus obtained showed characteristics similar to those of the drug sustained-release agent in Example 1. In addition, based on the porous carbon material obtained in this example, an adsorbent for adsorbing an organic matter thereon similar to that in Example 2 was obtained. The adsorbent for adsorbing an organic matter thereon thus obtained showed characteristic similar to those of the adsorbent for adsorbing an organic matter thereon in Example 2. Besides, the adsorbent obtained in this example showed characteristics similar to those of the adsorbent for adsorbing an allergen thereon in Example 3. Furthermore, based on the porous carbon material obtained in this example, a mask and an adsorption sheet similar to those in Example 4 could be obtained.

Example 7

Example 7 relates to the functional foods according to the first to third embodiments of the present invention. In Example 7, based on Porous Carbon Material A, Porous carbon Material B and Porous Carbon Material C described in Example 1, functional foods containing these porous carbon materials respectively were produced. Specifically, the functional foods were produced by a method in which a porous carbon material, a microcrystalline cellulose preparation obtained by coating microcrystalline cellulose with carboxymethyl cellulose sodium, a sweetening agent, and a seasoning agent are mixed and dispersed in water, followed by kneading and molding.

While the present invention has been described based on preferred examples, the invention is not limited to these examples, and various modifications are possible. The raw materials, manufacturing conditions and the like of the porous carbon materials in the examples are merely for exemplification, and they can be changed, as required.

Explanation of Reference Numerals

10 ... Main body part (Adsorption sheet) of mask for coping with pollinosis, 11 ... Non-woven fabric, 12 ... Porous carbon material

**Claims**

1. A drug sustained-release agent comprising a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

2. A drug sustained-release agent comprising a porous carbon material in which pores are arrayed in an arrangement corresponding to a crystal structure on a macroscopic basis.

3. A drug sustained-release agent comprising a porous carbon material in which pores are arrayed at a surface thereof in an arrangement corresponding to the (111) plane orientation of a face-centered cubic structure on a macroscopic basis.

4. The drug sustained-release agent according to any of claims 1 to 3, wherein a surface of the porous carbon material has undergone a chemical treatment or molecular modification.

5. The drug sustained-release agent according to any of claims 1 to 3, wherein a drug is adsorbed or supported on the porous carbon material in an amount of 1 to 200 parts by weight based on 100 parts by weight of the porous carbon material.

**6.** The drug sustained-release agent according to any of claims 1 to 3, wherein ibuprofen is adsorbed or supported on the porous carbon material as a drug.

**7.** An adsorbent for adsorbing an organic matter thereon, comprising a porous carbon material which includes spherical pores having an average diameter of $1\times10^{-9}$ to $1\times10^{-5}$ m and arrayed three-dimensionally and which has a surface area of not less than $3\times10^2$ $m^2/g$.

**8.** An adsorbent for adsorbing an organic matter thereon, comprising a porous carbon material in which pores are arrayed in an arrangement corresponding to a crystal structure on a macroscopic basis.

**9.** An adsorbent for adsorbing an organic matter thereon, comprising a porous carbon material in which pores are arrayed at a surface thereof in an arrangement corresponding to the (111) plane orientation of a face-centered cubic structure on a macroscopic basis.

**10.** The adsorbent according to any of claims 7 to 9, wherein a surface of the porous carbon material has undergone a chemical treatment or molecular modification.

**11.** The adsorbent according to any of claims 7 to 9, wherein the organic matter is indole.

**12.** The adsorbent according to any of claims 7 to 9, wherein the organic matter is creatinine.

**13.** The adsorbent according to any of claims 7 to 9, wherein the organic matter is uric acid.

**14.** The adsorbent according to any of claims 7 to 9, wherein the organic matter is adenosine.

**15.** The adsorbent according to any of claims 7 to 9, wherein the organic matter is lysozyme.

**16.** The adsorbent according to any of claims 7 to 9, wherein the organic matter is $\alpha$-amylase.

**17.** The adsorbent according to any of claims 7 to 9, wherein the organic matter is albumin.

**18.** The adsorbent according to any of claims 7 to 9, wherein the organic matter is 3-methylindole.

**19.** The adsorbent according to any of claims 7 to 9, wherein the organic matter is tryptophan.

**20.** The adsorbent according to any of claims 7 to 9, wherein the organic matter is indican.

**21.** The adsorbent according to any of claims 7 to 9, wherein the organic matter is theophylline.

**22.** The adsorbent according to any of claims 7 to 9, wherein the organic matter is inosine 5-monophosphate disodium salt.

**23.** The adsorbent according to any of claims 7 to 9, wherein the organic matter is adenosine 5-triphosphate disodium salt.

**24.** The adsorbent according to any of claims 7 to 9, wherein the organic matter is an organic manner having an average molecular weight of $1\times10^2$ to $5\times10^4$.

**25.** An adsorbent for medical use, comprising a porous carbon material which includes spherical pores having an average diameter of $1\times10^{-9}$ to $1\times10^{-5}$ m and arrayed three-dimensionally and which has a surface area of not less than $3\times10^2$ $m^2/g$.

**26.** An adsorbent for medical use, comprising a porous carbon material in which pores are arrayed in an arrangement corresponding to a crystal structure on a macroscopic basis.

**27.** An adsorbent for medical use, comprising a porous carbon material in which pores are arrayed at a surface thereof in an arrangement corresponding to the (111) plane orientation of a face-centered cubic structure on a macroscopic basis.

**28.** An adsorbent for oral administration, comprising a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

29. An adsorbent for oral administration, comprising a porous carbon material in which pores are arrayed in an arrangement corresponding to a crystal structure on a macroscopic basis.

30. An adsorbent for oral administration, comprising a porous carbon material in which pores are arrayed at a surface thereof in an arrangement corresponding to the (111) plane orientation of a face-centered cubic structure on a macroscopic basis.

31. An adsorbent for adsorbing an allergen thereon, comprising a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

32. An adsorbent for adsorbing an allergen thereon, comprising a porous carbon material in which pores are arrayed in an arrangement corresponding to a crystal structure on a macroscopic basis.

33. An adsorbent for adsorbing an allergen thereon, comprising a porous carbon material in which pores are arrayed at a surface thereof in an arrangement corresponding to the (111) plane orientation of a face-centered cubic structure on a macroscopic basis.

34. The adsorbent according to any of claims 31 to 33, wherein the allergen is an allergen arising from a mite.

35. The adsorbent according to any of claims 31 to 33, wherein the allergen is an allergen arising from pollen of Japanese cedar.

36. A functional food comprising a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

37. A functional food comprising a porous carbon material in which pores are arrayed in an arrangement corresponding to a crystal structure on a macroscopic basis.

38. A functional food comprising a porous carbon material in which pores are arrayed at a surface thereof in an arrangement corresponding to the (111) plane orientation of a face-centered cubic structure on a macroscopic basis.

39. A mask having an adsorbent comprising a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

40. A mask having an adsorbent comprising a porous carbon material in which pores are arrayed in an arrangement corresponding to a crystal structure on a macroscopic basis.

41. A mask having an adsorbent comprising a porous carbon material in which pores are arrayed at a surface thereof in an arrangement corresponding to the (111) plane orientation of a face-centered cubic structure on a macroscopic basis.

42. The mask according to any of claims 39 to 41, wherein a surface of the porous carbon material has undergone a chemical treatment or molecular modification.

43. An adsorption sheet comprising a sheet-shaped member and a support member for supporting the sheet-shaped member, the sheet-shaped member including a porous carbon material which includes spherical pores having an average diameter of $1 \times 10^{-9}$ to $1 \times 10^{-5}$ m and arrayed three-dimensionally and which has a surface area of not less than $3 \times 10^2$ m$^2$/g.

44. An adsorption sheet comprising a sheet-shaped member and a support for supporting the sheet-shaped member, the sheet-shaped member including a porous carbon material in which pores are arrayed in an arrangement corresponding to a crystal structure on a macroscopic basis.

**45.** An adsorption sheet comprising a sheet-shaped member and a support member for supporting the sheet-shaped member, the sheet-shaped member including a porous carbon material in which pores are arrayed at a surface thereof in an arrangement corresponding to the (111) plane orientation of a face-centered cubic structure on a macroscopic basis.

**46.** The adsorption sheet according to any of claims 43 to 45, wherein a surface of the porous carbon material has undergone a chemical treatment or molecular modification.

# F I G . 1

# F I G . 2

(A)

(B)

# FIG.3

# FIG.4

<table>
<tr><td colspan="2" align="center">**INTERNATIONAL SEARCH REPORT**</td><td colspan="2">International application No.<br>PCT/JP2009/064289</td></tr>
</table>

**A. CLASSIFICATION OF SUBJECT MATTER**

*A61K47/04*(2006.01)i, *A61K9/70*(2006.01)i, *A61K31/192*(2006.01)i, *A62B18/02*
(2006.01)i, *A62B23/02*(2006.01)i, *B01J20/20*(2006.01)i, *B01J20/28*(2006.01)i,
*C01B31/02*(2006.01)i

According to International Patent Classification (IPC) or to both national classification and IPC

**B. FIELDS SEARCHED**

Minimum documentation searched (classification system followed by classification symbols)
A61K47/04, A61K9/70, A61K31/192, A62B18/02, A62B23/02, B01J20/20,
B01J20/28, C01B31/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/MEDLINE/EMBASE/BIOSIS(STN), JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 62-084019 A (Mitsubishi Chemical Industries Ltd.),<br>17 April 1987 (17.04.1987),<br>claims; table 1<br>(Family: none) | 1,4,6<br>1-6 |
| X<br>Y | JP 2008-024611 A (Jellice Co., Ltd.),<br>07 February 2008 (07.02.2008),<br>claims<br>(Family: none) | 1,4,6<br>1-6 |
| Y | JP 2007-523108 A (Mattern, Udo),<br>16 August 2007 (16.08.2007),<br>claims; examples<br>& US 2005/0186272 A1 & EP 1566173 A1<br>& WO 2005/079759 A1 | 1-6 |

☒ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>13 November, 2009 (13.11.09) | Date of mailing of the international search report<br>24 November, 2009 (24.11.09) |
|---|---|
| Name and mailing address of the ISA/<br>   Japanese Patent Office | Authorized officer |
| Facsimile No. | Telephone No. |

Form PCT/ISA/210 (second sheet) (April 2007)

**INTERNATIONAL SEARCH REPORT**

| International application No. |
|---|
| PCT/JP2009/064289 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2005-343885 A  (Japan Science and Technology Agency),<br>15 December 2005 (15.12.2005),<br>entire text<br>& US 2006/0193919 A1 | 1-6 |
| Y | JP 2002-527335 A  (Allied-Signal Inc.),<br>27 August 2002 (27.08.2002),<br>examples; paragraph [0014]<br>& US 6261469 B1         & EP 1121334 A1<br>& WO 2000/021905 A1 | 1-46 |
| Y | Guan, G. et al, Preparation of carbon microparticle assemblies from phenolic resin using an inverse opal templating method,<br>Journal of Materials Science, 2007, 42(24),<br>pp.10196-10202 | 1-46 |
| Y | Take, H. et al, Preparation and electronic properties of nanoporous carbon inverse opal,<br>Japanese Journal of Applied Physics, 43(7A),<br>2004, pp.4453-4457 | 1-46 |
| X<br><br>Y | JP 2002-308785 A  (Kureha Chemical Industry Co., Ltd.),<br>23 October 2002 (23.10.2002),<br>claims<br>& US 2002/0176840 A1    & EP 1249241 A1 | 7,10-25,28,<br>31,34-36,39,<br>42,43,46<br>7-46 |
| X<br><br>Y | JP 2004-244414 A  (Meruku Hoei Kabushiki Kaisha),<br>02 September 2004 (02.09.2004),<br>claims<br>& US 2004/0141963 A1    & EP 1440692 A1<br>& KR 2004-0067916 A | 7,10-25,28,<br>31,34-36,39,<br>42,43,46<br>7-46 |
| X<br><br>Y | JP 62-223125 A  (Kureha Chemical Industry Co., Ltd.),<br>01 October 1987 (01.10.1987),<br>claims<br>& US 4761284 A          & DE 3023848 A1 | 7,10-25,28,<br>31,34-36,39,<br>42,43,46<br>7-46 |
| X<br><br><br>Y | JP 2007-169274 A  (Bluecher GmbH),<br>05 July 2007 (05.07.2007),<br>claims; paragraph [0033]<br>& US 2007/0141046 A1    & EP 1797889 A1 | 7,10-25,28,<br>31,34-36,39,<br>42,43,46<br>7-46 |

Form PCT/ISA/210 (continuation of second sheet) (April 2007)

**INTERNATIONAL SEARCH REPORT**

<table>
<tr><td>International application No.</td></tr>
<tr><td>PCT/JP2009/064289</td></tr>
</table>

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
The technical feature of the inventions in claims 1 to 46 of the present application is the utilization of a porous carbon material having a specific structure in various uses. However, the material *per se* has been well known by those skilled in the art (see, if required, JP 2002-527335 A, etc.) and, therefore, this technical feature cannot be considered as "a special technical feature" in the present invention.
Thus, it is considered that the "special technical feature" of the present invention is the uses of the aforesaid material. Therefore, the "special technical feature" is the uses of the aforesaid material as "a sustained releaser for drugs" in the inventions of (continued to extra sheet)

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.

PCT/JP2009/064289

Continuation of Box No.III of continuation of first sheet(2)

claims 1 to 6, as "an adsorbent" in the inventions of claims 7 to 35 and claims 39 to 46 and as "a functional food" in the inventions of claims 36 to 38. Taking the common technical knowledge at the point of the application into consideration, however, it cannot be recognized that there is a single general inventive concept among these uses. Such being the case, it should be concluded that the present international application does not comply with the requirement of unity of invention.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005262324 A
- JP SHO6211611 B
- JP 2005343885 A
- JP 3694305 B

**Non-patent literature cited in the description**

- **Anvar A. Zakhidov.** Carbon Structures with Three-Dimensional Periodicity at Optical Wavelengths. *Science,* 1998, 897